# EUROPEAN PATENT APPLICATION

(11) **EP 4 434 976 A1**
(43) Date of publication of application: **25.09.2024**
(21) Application number: 22895618.1
(22) Date of filing: 16.11.2022
(51) Int. Cl.: C07D 401/04, C07D 233/64

(54) **METHOD FOR PRODUCING 2-HETEROARYLPYRIDINE COMPOUND**

(30) Priority: 19.11.2021 JP 2021188981
(71) Applicant: Nippon Soda Co., Ltd., Tokyo 100-7010 (JP)
(72) Inventor: SUGAHARA, Erika, Takaoka-shi, Toyama 933-8507 (JP)
(74) Representative: Sonnenhauser, Thomas Martin
(86) International application number: PCT/JP2022/042474
(87) International publication number: WO 2023/090337

(57) **Abstract**

The present invention provides a method for producing a compound represented by formula (3) (in formula (3), Q, R¹ and R² are the same as those in formula (1) and formula (2)) comprising chemically reacting a compound represented by formula (1) (in the formula, Q represents a substituted or unsubstituted 5- to 6-membered or 9- to 10-membered heteroaryl group, and R¹ represents a C1-6 alkyl group) with a compound represented by formula (2) (in the formula, R² represents a hydrogen atom, a substituted or unsubstituted C1-6 alkyl group, a substituted or unsubstituted C3-6 cycloalkyl group, a substituted or unsubstituted phenyl group, or a substituted or unsubstituted 5- to 6-membered heteroaryl group, R^{a} represents a C1-6 alkyl group, and X⁻ represents a halide ion) in the presence of a base, and chemically reacting a product of the above chemical reaction with an ammonia or an ammonium salt.

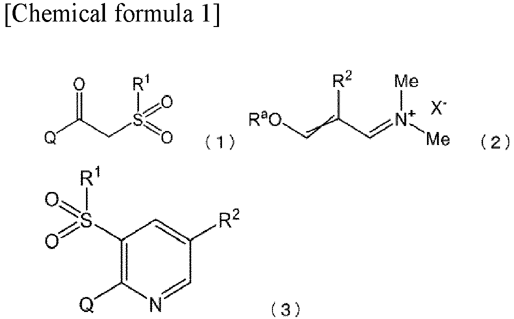

## Description

### [Technical Field]

The present invention relates to a method for producing a 2-heteroarylpyridine compound.

### [Background Art]

Nitrogen-containing heterocycles are a major partial structure (building blocks) constituting compounds that serve as active ingredients for pharmaceuticals and agricultural chemicals. In recent years, arthropod pest control agents having a pyridine ring have been actively developed. For example, Patent Documents 1 to 5 describe pyridine compounds having a 5-membered heteroaryl group and a sulfur-containing hydrocarbon group.

In addition, Non-Patent Document 1 discloses that a 3-phenyl-5-chloro-2-pyridone (4g) and a 3-hydroxycarbonyl-5-chloro-2-pyridone (4h) were produced by reacting a 2-chloro-N,N-dimethylamino trimethinium hexafluorophosphate with a methylphenylacetate and a 3-methoxy-3-oxopropanoic acid.

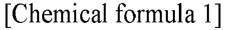

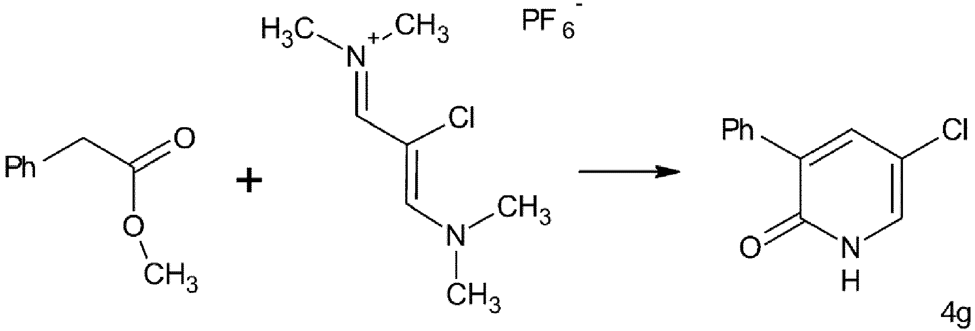

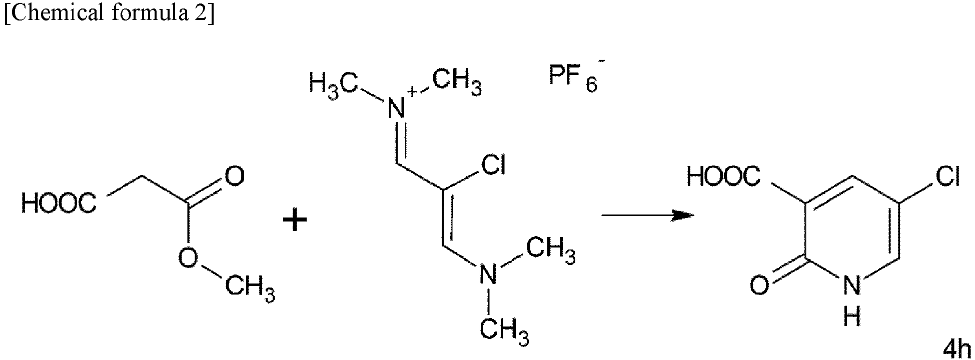

Patent Document 6 discloses a method for constructing a pyridine ring according to the following scheme.

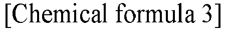

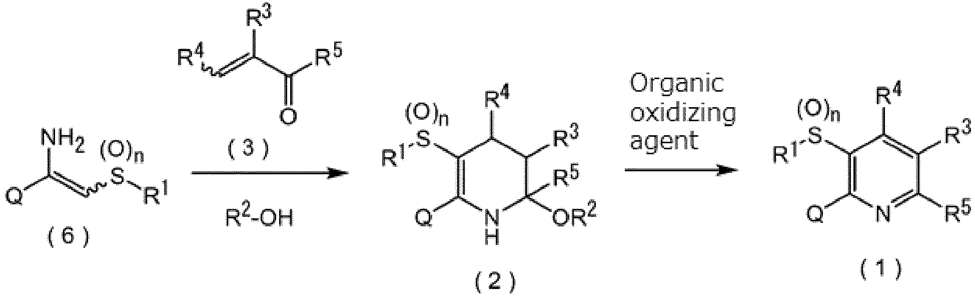

In the above scheme, Q represents a pyridyl group or the like, R¹ represents a C1-C6 alkyl group which may have one or more halogen atoms, R² represents a C1-C6 alkyl group or the like which may have one or more halogen atoms, R³ , R⁴ and R⁵ each represents a hydrogen atom, and n represents 0 or the like.

### [Citation List]

### [Patent Literature]

[Patent Document 1] WO 2017/016910 A
[Patent Document 2] WO 2017/050685 A
[Patent Document 3] WO 2020/050212 A
[Patent Document 4] WO 2020/071304 A
[Patent Document 5] WO 2020/090585 A
[Patent Document 6] WO 2018/194077 A

### [Non-Patent Literature]

[Non-Patent Document 1] Jean-Francuois Marcoux et al. "A General Preparation of Pyridines and Pyridones via the Annulation of Ketones and Esters" J. Org. Chem., Vol. 66, No. 12, 2001, 4194-4199.

### [Summary of Invention]

### [Technical Problem]

An object of the present invention is to provide a method for producing a 2-heteroarylpyridine compound having a sulfur-containing functional group, which is one of the building blocks.

### [Solution to Problem]

As a result of repeated studies to achieve the above object, the present invention including the following aspects has been completed.

[1] A method for producing a compound represented by formula (3) (hereinafter sometimes referred to as compound (3)), comprising:
   chemically reacting a compound represented by formula (1) (hereinafter sometimes referred to as compound (1)) with a compound represented by formula (2) (hereinafter sometimes referred to as compound (2)) in the presence of a base, and
   chemically reacting a product of the above chemical reaction with an ammonia or an ammonium salt. 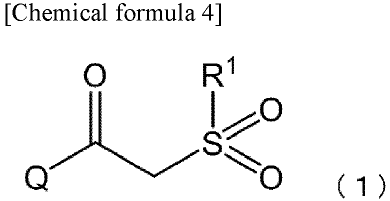
   (In formula (1), Q represents a substituted or unsubstituted 5- to 6-membered or 9- to 10-membered heteroaryl group, and R¹ represents a C1-6 alkyl group.) 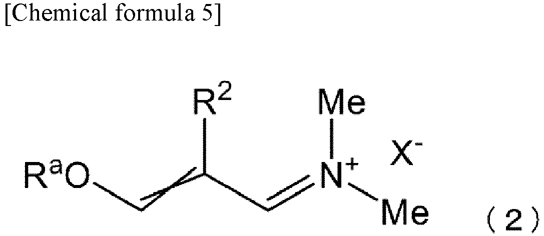
   (In formula (2), R² represents a hydrogen atom, a substituted or unsubstituted C1-6 alkyl group, a substituted or unsubstituted C3-6 cycloalkyl group, a substituted or unsubstituted phenyl group, or a substituted or unsubstituted 5- to 6-membered heteroaryl group, R^{a} represents a C1-6 alkyl group, and X⁻ represents a halide ion.) 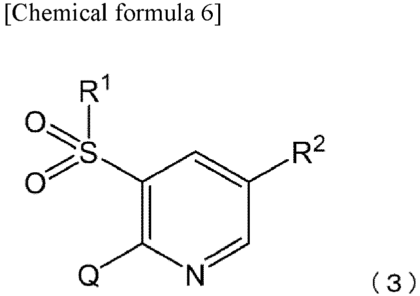
   (In formula (3), Q, R¹ and R² are the same as those in formula (1) and formula (2).)
[2] The method according to [1], wherein Q in formula (1) and formula (3) is a group represented by formula (4). 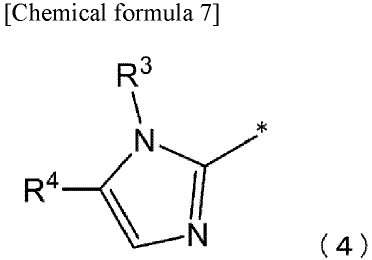 (In formula (4), * represents a bonding site, R³ represents a C1-6 alkyl group, R⁴ represents a hydrogen atom, a substituted or unsubstituted C1-6 alkyl group, a substituted or unsubstituted C2-6 alkenyl group or a 1,3-dioxolan-2-yl group.)
[3] A compound represented by formula (1a). 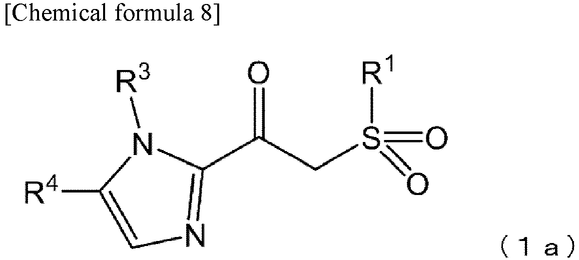 (In formula (1a), R¹ represents a C1-6 alkyl group, R³ represents a C1-6 alkyl group, R⁴ represents a hydrogen atom, a substituted or unsubstituted C1-6 alkyl group, a substituted or unsubstituted C2-6 alkenyl group or a 1,3-dioxolan-2-yl group.)

### [Advantageous Effects of Invention]

According to the production method of the present invention, a compound represented by formula (3) can be obtained in high yield.

### [Description of Embodiments]

In the present invention, the term "unsubstituted" refers to a group having only a mother nucleus. When "substituted" is not mentioned and only the name of the mother nucleus is described, it means "unsubstituted" unless otherwise specified.

On the other hand, the term "substituted" means that any hydrogen atom of the group serving as a mother nucleus is substituted with a group (substituent) having the same or different structure from the mother nucleus. Therefore, a "substituent" is another group bonded to the mother nucleus. The number of substituents may be one, or two, or more. Two or more substituents may be the same or different.

A term such as "C1-6" indicates that the number of carbon atoms in the group serving as a mother nucleus is 1 to 6. This number of carbon atoms does not include the number of carbon atoms present in the substituents. For example, a butyl group having an ethoxy group as a substituent is classified as a C2 alkoxy C4 alkyl group.

The "substituent" is not particularly limited as long as it is chemically permissible and has the effects of the present invention.

Examples of the groups that can be the "substituent" are shown below.
C1-6 alkyl groups such as a methyl group, an ethyl group, an n-propyl group, an i-propyl group, an n-butyl group, an s-butyl group, an i-butyl group, a t-butyl group, an n-pentyl group, an n-hexyl group or the like;
C2-6 alkenyl groups such as a vinyl group, a 1-propenyl group, a 2-propenyl group (allyl group), a 1-butenyl group, a 2-butenyl group, a 3-butenyl group, a 1-methyl-2-propenyl group, a 2-methyl-2-propenyl group or the like;
C2-6 alkynyl groups such as an ethynyl group, a 1-propynyl group, a 2-propynyl group, a 1-butynyl group, a 2-butynyl group, a 3-butynyl group, a 1-methyl-2-propynyl group or the like;
C3-6 cycloalkyl groups such as a cyclopropyl group, a cyclobutyl group, cyclopentyl group, a cyclohexyl group or the like;
a phenyl group;
3- to 6-membered heterocyclyl groups;
C1-6 alkoxy groups such as a methoxy group, an ethoxy group, an n-propoxy group, an i-propoxy group, an n-butoxy group, an s-butoxy group, an i-butoxy group, a t-butoxy group or the like;
C6-10 aryloxy groups such as a phenoxy group, a naphthoxy group or the like;
5- to 6-membered heteroaryloxy groups such as a thiazolyloxy group, a pyridyloxy group or the like;
C1-6 alkoxycarbonyl groups such as a methoxycarbonyl group, an ethoxycarbonyl group, an n-propoxycarbonyl group, an i-propoxycarbonyl group, an n-butoxycarbonyl group, a t-butoxycarbonyl group or the like;
halogeno groups such as a fluoro group, a chloro group, a bromo group, an iodo group or the like;
C1-6 haloalkyl groups such as a chloromethyl group, a chloroethyl group, a trifluoromethyl group, a 1,2-dichloro-n-propyl group, a 1-fluoro-n-butyl group, a perfluoro-n-pentyl group or the like;
C1-6 haloalkoxy groups such as a trifluoromethoxy group, a 2-chloro-n-propoxy group, a 2,3-dichlorobutoxy group or the like;
a formylamino group;
C1-6 alkylcarbonylamino groups such as an acetylamino group, a propanoylamino group, a butyrylamino group, an i-propylcarbonylamino group or the like;
C1-6 alkoxycarbonylamino groups such as a methoxycarbonylamino group, an ethoxycarbonylamino group, an n-propoxycarbonylamino group, an i-propoxycarbonylamino group or the like;
unsubstituted or substituted aminocarbonyl groups such as an aminocarbonyl group, a dimethylaminocarbonyl group, a phenylaminocarbonyl group, an N-phenyl-N-methylaminocarbonyl group or the like;
C1-6 alkylthio groups such as a methylthio group, an ethylthio group, an n-propylthio group, an i-propylthio group, an n-butylthio group, an i-butylthio group, an s-butylthio group, a t-butylthio group or the like;
C1-6 haloalkylthio groups such as a trifluoromethylthio group, a 2,2,2-trifluoroethylthio group or the like;
C1-6 alkylsulfonyl groups such as a methylsulfonyl group, an ethylsulfonyl group, a t-butylsulfonyl group or the like;
C1-6 haloalkylsulfonyl groups such as a trifluoromethylsulfonyl group, a 2,2,2-trifluoroethylsulfonyl group or the like;
a cyano group; a nitro group;

Moreover, any hydrogen atom in these "substituents" may be substituted with a group having a different structure. Examples of the substituent in this case include a C1-6 alkyl group, a C1-6 haloalkyl group, a C1-6 alkoxy group, a C1-6 haloalkoxy group, a halogeno group, a cyano group and a nitro group.

Furthermore, the above-mentioned "3- to 6-membered heterocyclyl group" includes 1 to 4 heteroatoms selected from the group consisting of a nitrogen atom, an oxygen atom and a sulfur atom as a ring constituent atom. The heterocyclyl group may be monocyclic or polycyclic. In the polycyclic heterocyclyl group, as long as at least one ring is a heterocyclic ring, the remaining rings may be any of a saturated alicyclic ring, an unsaturated alicyclic ring or an aromatic ring. Examples of the "3- to 6-membered heterocyclyl group" include a 3- to 6-membered saturated heterocyclyl group, a 5- to 6-membered heteroaryl group, a 5- to 6-membered partially unsaturated heterocyclyl group and the like.

Examples of the 3- to 6-membered saturated heterocyclyl group include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, a dioxanyl group and the like.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group and the like.

Examples of the 6-membered heteroaryl group include a pyridyl group, a pyrazinyl group, a pyrimidinyl group, a pyridazinyl group, and a triazinyl group.

Examples of the 5-membered partially unsaturated heterocyclyl group include a pyrrolinyl group, a dihydrofuranyl group, an imidazolinyl group, a pyrazolinyl group, an oxazolinyl group, an isoxazolinyl group and the like.

Examples of the 6-membered partially unsaturated heterocyclyl group include a dihydropyranyl group and the like.

### <Method for producing compound (3)>

The method for producing compound (3) according to the present invention includes chemically reacting compound (1) with compound (2) in the presence of a base (hereinafter sometimes referred to as the first reaction), and chemically reacting the product of the first reaction with an ammonia or an ammonium salt (hereinafter sometimes referred to as the second reaction).

### (Compound (1))

Compound (1) is represented by formula (1).

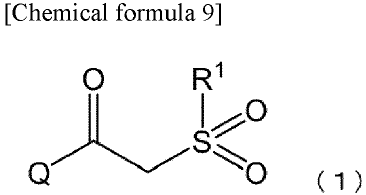

In formula (1), R¹ represents a C1-6 alkyl group.

The C1-6 alkyl group for R¹ may be linear or branched. Examples of the C1-6 alkyl group for R¹ include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, an i-hexyl group and the like.

In formula (1), Q represents a substituted or unsubstituted 5- to 6-membered or 9- to 10-membered heteroaryl group.

Examples of the "5-membered heteroaryl group" include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group and the like.

Examples of the "6-membered heteroaryl group" include a pyridyl group, a pyrazinyl group, a pyrimidinyl group, a pyridazinyl group, a triazinyl group and the like.

Examples of the "9-membered heteroaryl group" include an indolyl group, an isoindolyl group, a benzofuranyl group, a benzothienyl group, an indazolyl group, a benzimidazolyl group, a benzoxazolyl group, a benzisoxaozolyl group, a benzothiazolyl group, a benzisothiazolyl group and the like.

Examples of the "10-membered heteroaryl group" include an isoquinolinyl group, a quinazolinyl group, a 1,2,4-benzotriazinyl group, a 1,2,3,4-benzotetrazinyl group, and the like.

Substituents on the "5- to 6-membered or 9- to 10-membered heteroaryl group" for Q include halogeno groups such as a fluoro group, a chloro group, a bromo group, an iodo group or the like; C1-6 alkyl groups such as a methyl group, an ethyl group, an n-propyl group, an i-propyl group, an n-butyl group, an s-butyl group, an i-butyl group, a t-butyl group, an n-pentyl group, an n-hexyl group or the like; C1-6 haloalkyl groups such as a difluoromethyl group, a trifluoromethyl group, a perfluoroethyl group, a 1,2,2,3,3,3-hexafluoropropyl group, a perfluoropropyl group, a 1,2,3,3,3-pentafluoro-2-(trifluoromethyl)propyl group or the like; C2-6 alkynyl groups such as an ethynyl group, a 1-propynyl group, a 2-propynyl group, a 1-butynyl group, a 2-butynyl group, a 3-butynyl group, a 1-methyl-2-propynyl group or the like; C2-6 haloalkynyl groups such as a 3,3,4,4,4-pentafluorobut-1-en-1-yl group, a 2-chloro-3,3,3-trifluoroprop-1-en-1-yl group, a 2,2-dichloroethynyl group, a 2,3,3,4,4,4-hexafluorobut-1-en-1-yl group, a 2-chloro-3,3,4,4,4-pentafluorobut-1-en-1-yl group or the like; C1-6 alkoxy groups such as a methoxy group, an ethoxy group, an n-propoxy group, an i-propoxy group, an n-butoxy group, an s-butoxy group, an i-butoxy group, a t-butoxy group or the like; C1-6 haloalkoxy groups such as a difluoromethoxy group, a trifluoromethoxy group, a 2,2-difluoroethyl group, a 2,2,2-trifluoroethyl group or the like; a phenyl group; phenyl groups substituted with a halogeno group, a C1-6 alkyl group, a C1-6 haloalkyl group or a C1-6 haloalkoxy group, such as a 4-chlorophenyl, a 4-methylphenyl group, a 4-trifluoromethylphenyl group, a 4-trifluoromethoxyphenyl group or the like; a formyl group, a dimethoxymethyl group, a diethoxymethyl group, a 1,3-dioxolan-2-yl group or a cyano group; and the like.

In the present invention, Q is preferably a group represented by formula (4).

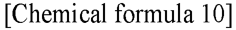

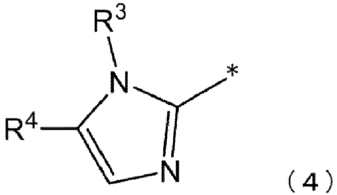

(In formula (4), * represents a bonding site, R³ represents a C1-6 alkyl group, R⁴ represents a hydrogen atom, a substituted or unsubstituted C1-6 alkyl group, a substituted or unsubstituted C2-6 alkenyl group or a 1,3-dioxolan-2-yl group.)

Specifically, compound (1) is preferably a compound represented by formula (1a) (hereinafter sometimes referred to as compound (1a)).

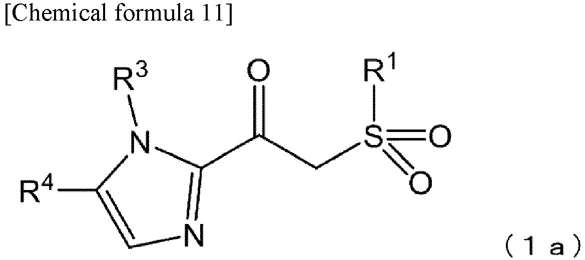

In formula (1a), R¹ represents a C1-6 alkyl group, R³ represents a C1-6 alkyl group, R⁴ represents a hydrogen atom, a substituted or unsubstituted C1-6 alkyl group, a substituted or unsubstituted C2-6 alkenyl group or a 1,3-dioxolan-2-yl group.

Compound (1a) is a novel compound and is useful as a substrate in the production method of the present invention.

The C1-6 alkyl group for R³ and R⁴ can be the same as that for R¹.

Examples of the C2-6 alkenyl group for R⁴ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group and the like.

Substituents on the "C1-6 alkyl group" and the "C2-6 alkenyl group" for R⁴ include halogeno groups such as a fluoro group, a chloro group, a bromo group, an iodo group or the like; a hydroxyl group; C1-6 alkoxy groups such as a methoxy group, an ethoxy group, an n -propoxy group, an i-propoxy group, an n-butoxy group, an s-butoxy group, an i-butoxy group, a t-butoxy group or the like; C1-6 haloalkoxy groups such as a 2-chloro-n-propoxy group, a 2,3-dichloro butoxy group, a trifluoromethoxy group or the like; a phenyl group; phenyl groups substituted with a halogeno group, a C1-6 haloalkyl group or a C1-6 haloalkyl group, such as a 4-chlorophenyl group, a 4-trifluoromethylphenyl group, a 4-trifluoromethoxyphenyl group or the like; and a cyano group. Among these, halogeno groups are preferred.

Examples of the halogeno-substituted C1-6 alkyl group for R⁴ include a fluoromethyl group, a difluoromethyl group, a trifluoromethyl group, a 2,2,2-trifluoroethyl group, a pentafluoroethyl group, a 3,3,3-trifluoro propyl group, a 2,2,3,3,3-pentafluoropropyl group, a 1-chloro-2,2,3,3,3-pentafluoropropyl group, a 1,2,2,3,3,3-hexafluoropropyl group, a perfluoropropyl group, a 2,2,2-trifluoro-1-trifluoromethylethyl group, a perfluoroisopropyl group, a 4-fluorobutyl group, a 1,4,4,4-tetrafluorobutyl group, a 2,2,3,3,4,4,4-heptafluorobutyl group, a 1,2,2,3,3,4,4,4-octafluorobutyl group, a perfluorobutyl group, a 3,3,3-trifluoro-2-trifluoromethylpropyl group, a 1-chloro-2,3,3,3-tetrafluoro-2-trifluoromethylpropyl group, a 1,2,3,3,3-pentafluoro-2-trifluoromethylpropyl group, a perfluoropentyl group, a perfluorohexyl group and the like.

Examples of the halogeno-substituted C2-6 alkenyl group for R⁴ include C2-6 haloalkenyl groups such as a 2,3,3,3-tetrafluoro-1-propenyl group, a 3,3,3-trifluoro-1-propenyl group, a 2-chloro-3, 3,3-trifluoro-1-propenyl group, a 2-bromo-3,3,3-trifluoro-1-propenyl group, a 3,3,3-trifluoro-2-trifluoromethyl-1-propenyl group, a 3,3,4,4,4-pentafluoro-1-butenyl group, a 2,3,3,4,4,4-hexafluoro-1-butenyl group, a 2-chloro-3,3,4,4, 4-pentafluoro-1-butenyl group or the like, and the like.

R⁴ is preferably a 2-chloro-3,3,3-trifluoro-1-propenyl group. The stereoisomerism of the 2-chloro-3,3,3-trifluoro-1-propenyl group may be a mixture of E form/Z form, or may be a Z form alone or an E form alone.

Compound (1) used in the present invention may be one synthesized using a known method, or may be a commercially available one. Specifically, the following compounds can be mentioned.

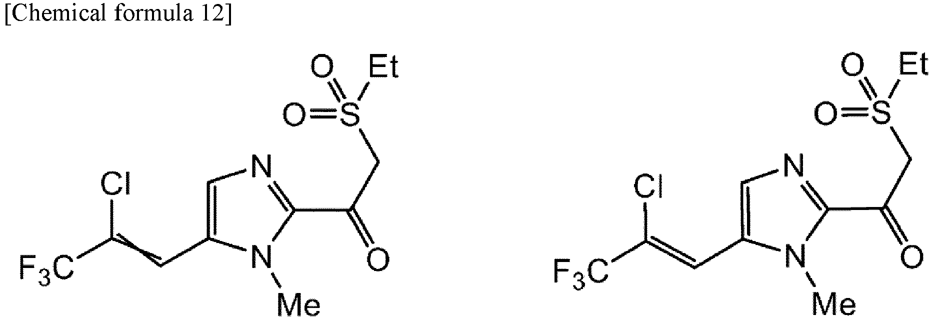

The following notation in the chemical formula is an undefined double stereo bond.

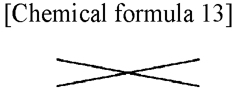

### (Compound (2))

Compound (2) is represented by formula (2).

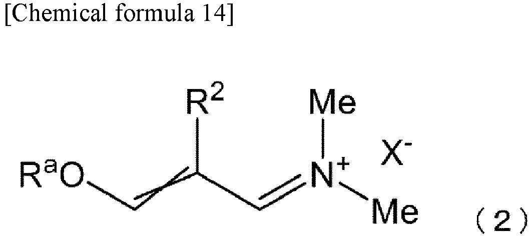

In formula (2), R² is a hydrogen atom, substituted or unsubstituted C1-6 alkyl group, a substituted or unsubstituted C3-6 cycloalkyl group, a substituted or unsubstituted phenyl group, or a substituted or unsubstituted 5- to 6-membered heteroaryl group.

The C1-6 alkyl group for R² can be the same as the C1-6 alkyl group for R¹.

Examples of the substituents on the "C1-6 alkyl group" for R² include halogeno groups such as a fluoro, a chloro, a bromo, an iodo group or the like; a hydroxyl group; C1-6 alkoxy groups such as a methoxy group, an ethoxy group, an n-propoxy group, an i-propoxy group, an n-butoxy group, an s-butoxy group, an i-butoxy group, a t-butoxy group or the like; C1-6 haloalkoxy groups such as a 2-chloro-n-propoxy group, a 2,3-dichlorobutoxy group, a trifluoromethoxy group or the like; a phenyl group; phenyl groups substituted with a halogeno group, a C1-6 haloalkyl group or a C1-6 haloalkoxy group, such as a 4-chlorophenyl group, a 4-trifluoromethylphenyl group, a 4-trifluoromethoxyphenyl group or the like; and a cyano group. Among these, a halogeno group is preferable.

Examples of the halogeno-substituted C1-6 alkyl group for R² include a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a 1-chloro-2,2,3,3,3-pentafluoropropyl group, a 1,2,2,3,3,3-hexafluoropropyl group, a perfluoropropyl group, a 2,2,2-trifluoro-1-trifluoromethylethyl group, a perfluoroisopropyl group, a 4-fluorobutyl group, a 1,4,4,4-tetrafluorobutyl group, a 2,2,3,3,4,4,4-heptafluorobutyl group, a 1,2,2,3,3,4,4,4-octafluorobutyl group, a perfluorobutyl group, a 3,3,3-trifluoro-2-trifluoromethylpropyl group, a 1-chloro-2,3,3,3-tetrafluoro-2-trifluoromethylpropyl group, a 1,2,3,3,3-pentafluoro-2-trifluoromethylpropyl group, a perfluoropentyl group, a perfluorohexyl group and the like.

Examples of the C3-6 cycloalkyl group for R² include a cyclopropyl group, a cyclobutyl group, a cyclopentyl group, a cyclohexyl group and the like.

The 5- to 6-membered heteroaryl group for R² is a 5- or 6-membered ring containing 1, 2, 3, or 4 heteroatoms selected from the group consisting of a nitrogen atom, an oxygen atom and a sulfur atom as a ring-constituting atom. When there are two or more heteroatoms, they may be the same or different.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group and the like.

Examples of the 6-membered heteroaryl group include a pyridyl group, a pyrazinyl group, a pyrimidinyl group, a pyridazinyl group, a triazinyl group and the like.

Examples of the substituents on the "C3-6 cycloalkyl group", the "phenyl group", or the "5-6 membered heteroaryl group" for R² include halogeno groups such as a fluoro group, a chloro group, a bromo group, an iodo group or the like; C1-6 alkyl groups such as a methyl group, an ethyl group, an n-propyl group, an i-propyl group, an n-butyl group, an s-butyl group, an i-butyl group, a t-butyl group, an n-pentyl group, an n-hexyl group or the like; C1-6 haloalkyl groups such as a chloromethyl group, a chloroethyl group, a trifluoromethyl group, a 1,2-dichloro-n-propyl group, a 1-fluoro-n-butyl group or the like; a hydroxyl group; C1-6 alkoxy groups such as a methoxy group, an ethoxy group, an n-propoxy group, an i-propoxy group, an n-butoxy group, an s-butoxy group, an i-butoxy group, a t-butoxy group or the like; C1-6 haloalkoxy groups such as a 2-chloro-n-propoxy group, a 2,3-dichloro a butoxy group, trifluoromethoxy group or the like; and a cyano group.

In formula (2), R^{a} represents a C1-6 alkyl group. Examples of the C1-6 alkyl group for R^{a} include the same groups as for R¹.

In formula (2), X⁻ represents a halide ion. Examples of the halide ion for X⁻ include a fluoride ion, a chloride ion, a bromide ion, and an iodide ion.

Compound (2) used in the present invention may be one synthesized using a known method, for example, the method described in WO 2020/009132A, or may be a commercially available one.

For example, compound (2) can be synthesized using the scheme shown below.

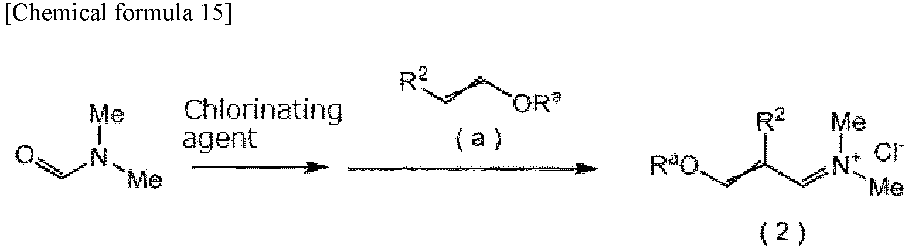

That is, the method is one in which compound (a) is reacted with dimethylformamide and a chlorinating agent.

As the chlorinating agent, any chlorinating agent may be used as long as it reacts with dimethylformamide to produce a Vilsmeier reagent, and examples thereof include a chlorine, a phosgene, an oxalyl chloride, a thionyl chloride, a phosphorus oxychloride and the like. R² and R^{a} in compound (a) are the same as in formula (2).

Moreover, compound (b) can be used in the same manner instead of compound (a).

R² and R^{a} in compound (b) are the same as in formula (2).

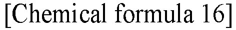

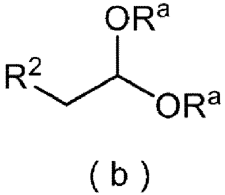

Although the amount of compound (2) to be used is not particularly limited, it is, for example, 1.0 to 5.0 mol, and preferably 1.0 to 2.0 mol, with respect to 1 mol of compound (1).

Examples of the base present in the first reaction include inorganic bases such as a sodium carbonate, a sodium hydrogen carbonate, a potassium carbonate, a potassium hydrogen carbonate, a sodium hydroxide, a potassium hydroxide, a sodium hydride, a potassium hydride or the like; and organic bases such as a triethylamine, a tetramethylethylenediamine, an N,N-diisopropylamine, an N,N-dimethylaniline, an N,N-diethylaniline, a 4-methylmorpholine, a 1-azabicyclo[2.2.2]octane, a 1,4-diazabicyclo[2.2.2]octane (abbreviation: DABCO), a 1,8-diazabicyclo[5.4.0]undec-7-ene, a 1,5-diazabicyclo[4.3.0]non-5-ene, a pyridine, a 4-(dimethylamino)pyridine, a 2,6-dimethylpyridine or the like. Among these, organic bases are preferable.

Although the amount of the base to be used is not particularly limited, it is, for example, 1.0 to 10.0 mol with respect to 1 mol of compound (1).

Due to the coexistence of the inorganic base and compound (1), some or all of compound (1) may change to compound (1') (enolate-formation), but this has no effect on the first reaction.

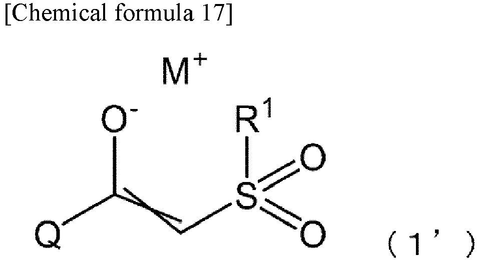

In formula (1'), M⁺ represents a counter cation, and Q and R¹ represent the same as those in formula (1).

The first reaction and the second reaction can be performed without a solvent or in a solvent.

If compound (1) or compound (1') is liquid at the reaction temperature, a solvent may not be used. Considering operability, it is preferable to carry out the reaction in an organic solvent. The organic solvent is not particularly limited as long as it is inert to compound (1) or compound (1'). Examples of the organic solvent include ethers such as a diethyl ether, a diisopropyl ether, a diethylene glycol dimethyl ether (product name: Diglyme), a tetrahydrofuran (abbreviation: THF) or the like; halogenated hydrocarbons such as a dichloromethane, a chloroform, a 1,2-dichloroethane (abbreviation: DCE) or the like; aliphatic hydrocarbons such as a pentane, a hexane, a cyclohexane, a heptane, an octane or the like; aromatic hydrocarbons such as a toluene, a xylene or the like; aprotic polar solvents such as an N,N-dimethylformamide (abbreviation: DMF), an N,N'-dimethylpropylene urea (abbreviation: DMPU), a hexamethylphosphoric acid triamide (abbreviation: HMPA) or the like; an acetonitrile; and the like. Although the amount of the organic solvent to be used is not particularly limited, it is preferably 10 to 500 parts by weight with respect to 1 part by weight of compound (1) or compound (1').

Although the details are unknown, it is presumed that the first reaction produces compounds represented by formulas (5), (6), (7) and the like.

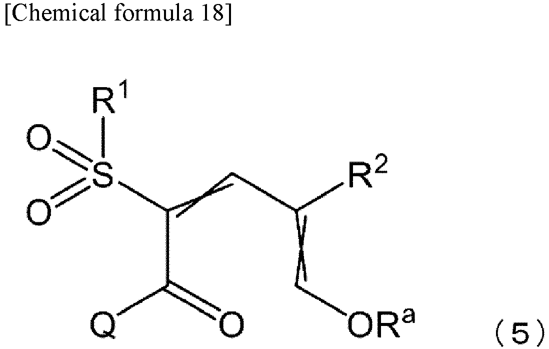

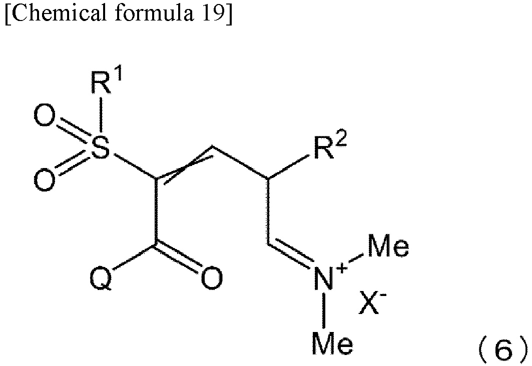

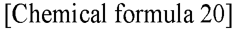

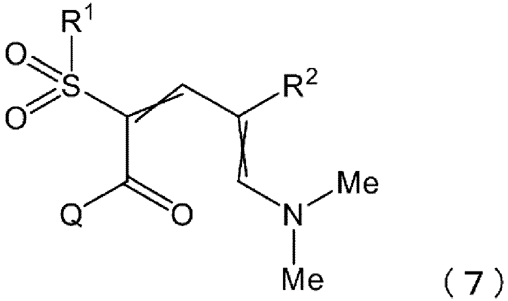

In formulas (5), (6), and (7), Q, R¹ and R² are the same as those in formulas (1) and (2).

As the ammonia, ammonia gas and ammonia water can be used. Examples of the ammonium salt include an ammonium chloride, an ammonium acetate, an ammonium carbonate, an ammonium sulfate, an ammonium nitrate and the like.

Although the amount of the ammonia or the ammonium salt to be used is not particularly limited, it is, for example, 1.0 to 5.0 mol, and preferably 1.5 to 3.0 mol with respect to 1 mol of compound (1).

The order of addition of compound (1), compound (2), a base and optionally an organic solvent to the first reaction system is not particularly limited.

The order of addition of the product of the first reaction, the ammonia or the ammonium salt, and optionally the organic solvent to the second reaction system is not particularly limited.

One embodiment includes obtaining a mixed solution by mixing compound (1) and a base together in an organic solvent or without a solvent, completing the first reaction by adding compound (2) to the mixed solution and stirring, followed by completing the second reaction by adding an ammonia or an ammonium salt to a liquid containing the product of the first reaction and stirring.

Each substance may be added in its entirety at once or in small amounts gradually.

The reaction may be carried out in such a manner that the first reaction and the second reaction are carried out sequentially in a single reactor, or may be carried out in such a manner that the first reaction is carried out in a first reactor, and the product of the first reaction is transferred to a second reactor to carry out the second reaction in the second reactor.

After the first reaction is completed, extraction is performed with an organic solvent, and the resulting organic layer is subjected to post-treatments such as drying and concentration. Furthermore, if necessary, the product of the first reaction may be purified by recrystallization, chromatography or the like.

The temperature during the first reaction is not particularly limited, and is, for example, 0°C to 50°C. The pressure during the first reaction is not particularly limited, and is, for example, 0.1 to 1 MPa, and preferably 0.1 to 0.5 MPa. The reaction time is not particularly limited, and is, for example, 0.5 to 24 hours. The first reaction is preferably carried out under an inert gas atmosphere.

The temperature during the second reaction can be selected as appropriate, and is for example, 50°C to 120°C. To obtain a high temperature, heating may be carried out under reflux. In the heating under reflux, the low-boiling substances generated in the first reaction or the like may be distilled off. The pressure during the second reaction is, for example, 0.1 to 1 MPa, preferably 0.1 to 0.5 MPa. An organic solvent can be used in the second reaction. The organic solvent used may be replaced from one with a low boiling point to one with a high boiling point depending on the reaction temperature. The time for the second reaction is, for example, 1 hour to 24 hours. The second reaction is preferably carried out under an inert gas atmosphere.

### (Compound (3))

Compound (3) obtained by the production method of the present invention is represented by formula (3).

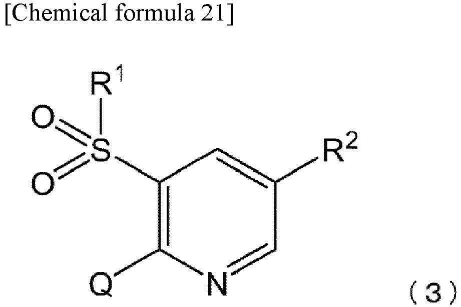

In formula (3), Q, R¹ and R² are the same as those in formula (1) and formula (2).

After the completion of the second reaction, post-treatments such as extraction with an organic solvent and drying and concentration of the resulting organic layer are performed. Further, if necessary, purification can be performed by recrystallization, chromatography or the like.

### [EXAMPLES]

The present invention will be described below in more detail with reference to the Examples. Note that the present invention is not limited to the following Examples.

### [Reference Example 1]

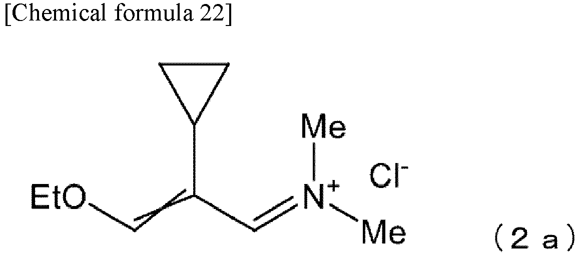

### Synthesis of 2-cyclopropyl-3-ethoxy-N,N-dimethyl-propan-2-en-1-iminium chloride (formula (2a))

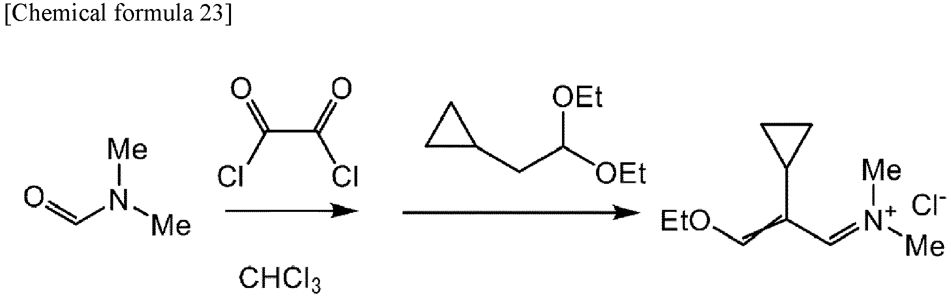

A mixture of N,N-dimethylformamide (0.22 g) and chloroform (1 mL) was added dropwise to a mixture of oxalyl chloride (0.38 g) and chloroform (3 mL) under ice cooling. After the dropwise addition was completed, the resulting mixture was stirred at room temperature for 30 minutes. The resulting liquid was cooled to 0°C. A mixed solution of (2,2-diethoxyethyl)cyclopropane (0.16 g) and chloroform (1 mL) was added to this solution, and the resulting mixture was stirred for 2 hours under heating reflux to obtain a solution containing the target compound (hereinafter, referred to as solution (2a)).

### [Reference Example 2]

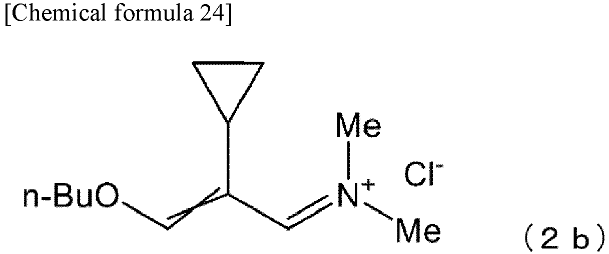

### Synthesis of N-(3-butoxy-2-cyclopropylallylidene)-N-methylmethanaminium chloride (formula (2b))

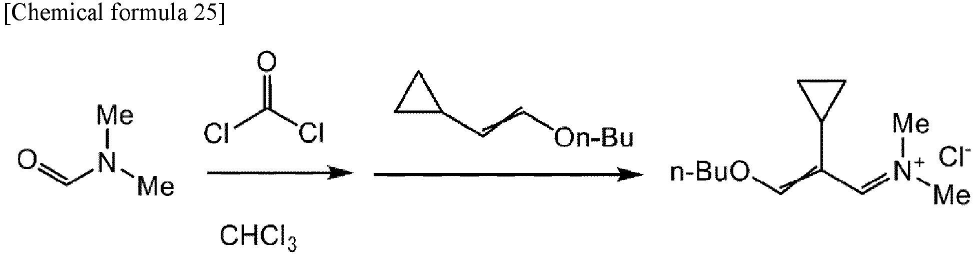

Phosgene (14.8 g) was blown into a mixture of N,N-dimethylformamide (8.01 g) and chloroform (150 mL) under ice cooling. After the blowing was completed, the resulting mixture was stirred for 30 minutes under ice cooling. From this reaction mixture, 50 mL of chloroform was distilled off under reduced pressure and phosgene gas was expelled. The obtained vacuum distillation residue was heated to 65°C.

(2-Butoxyvinyl)cyclopropane (13.82 g) was added dropwise to this liquid over 30 minutes, and the resulting mixture was stirred at 65°C for 2 hours.

The resulting reaction mixture was concentrated under reduced pressure to obtain 23.34 g of a residue containing the target compound (compound represented by formula (2b)).

### [Example 1]

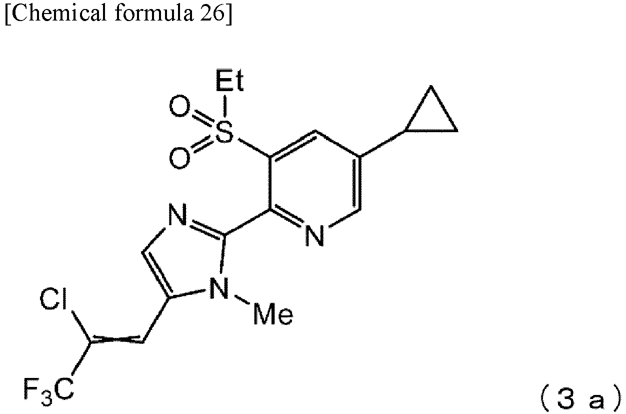

2-(5-(2-chloro-3,3,3-trifluoroprop-1-en-1-yl)-1-methyl-1H-imidazol-2-yl)-5-cyclopropyl-3-(ethylsulfonyl)pyridine (formula (3a)) was produced using the following procedure.

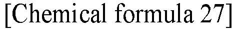

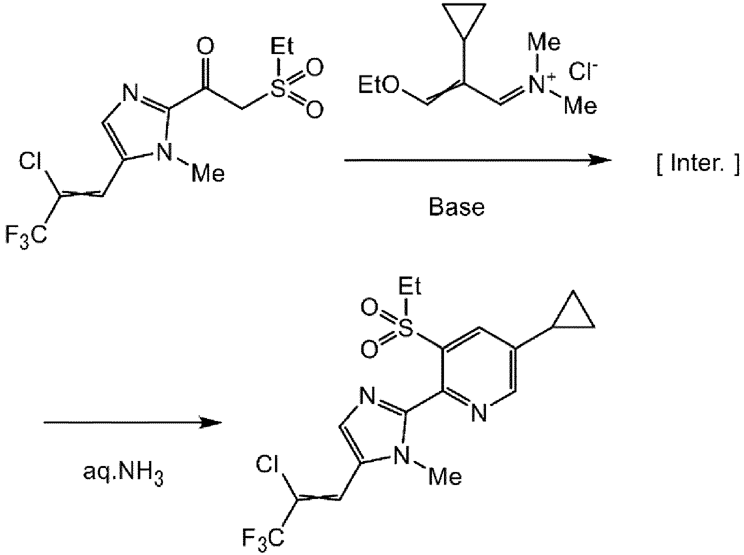

Solution (2a) was cooled to 0°C. To this was added a mixture of triethylamine (0.84 mL), 1-[5-(2-chloro-3,3,3-trifluoroprop-1-en-1-yl)-1-methylimidazol-2-yl]-2-(ethylsulfonyl)ethane-1-one (0.34g) and chloroform (3 mL). Thereafter, the resulting mixture was stirred at room temperature for 9 hours.

The resulting liquid was cooled to 0°C. Then, ethanol (5 mL) and 28% aqueous ammonia (0.18 mL) were added, and the resulting mixture was stirred at 30°C for 3 hours.

Water was added to the obtained liquid, and the resulting mixture was extracted with chloroform. The organic phase was dried over anhydrous magnesium sulfate and concentrated under reduced pressure to obtain the target compound in a yield of 47%.

The intermediate (Inter.) is presumed to be compounds represented by the chemical formulas shown below.

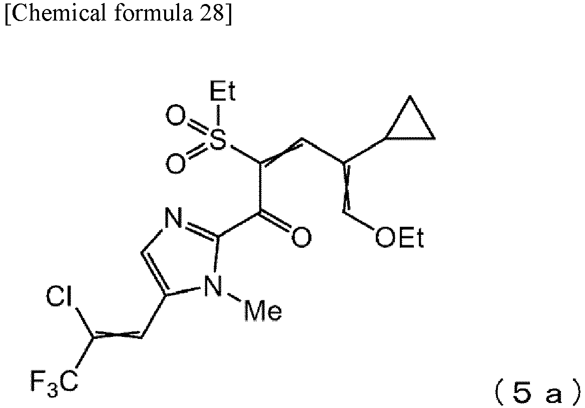

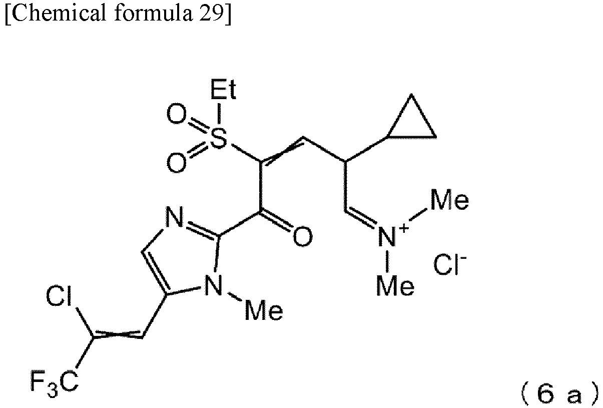

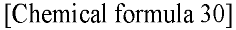

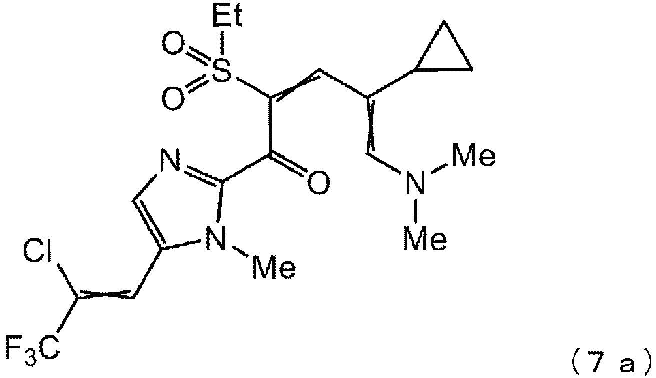

### [Example 2]

A compound represented by formula (3a) was obtained in the same manner as in Example 1, except that the compound represented by formula (2b) obtained in Reference Example 2 was used instead of the compound represented by formula (2a).

In this case, the intermediate (Inter.) is presumed to be compounds represented by the above formula (6a) and formula (7a), and a compound represented by the following formula (5b).

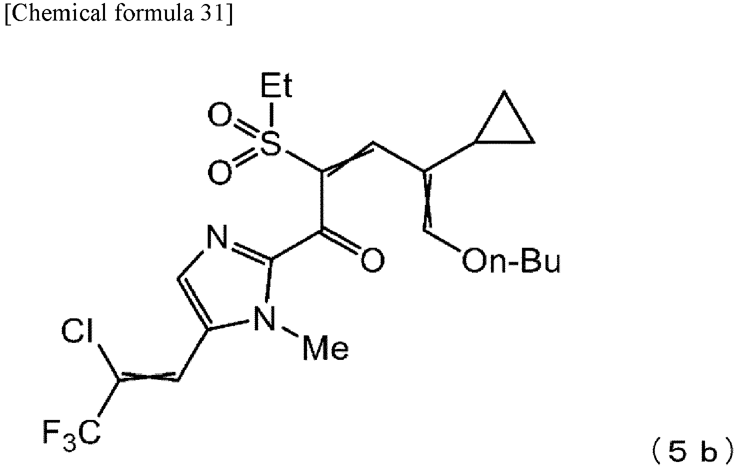

### [Industrial Applicability]

The production method of the present invention can efficiently obtain a 2-heteroarylpyridine compound having a sulfur-containing functional group, which is a major partial structure constituting compounds that serve as active ingredients for pharmaceuticals and agricultural chemicals.

## Claims

1. A method for producing a compound represented by formula (3), comprising:
chemically reacting a compound represented by formula (1) with a compound represented by formula (2) in the presence of a base, and
chemically reacting a product of the above chemical reaction with an ammonia or an ammonium salt, 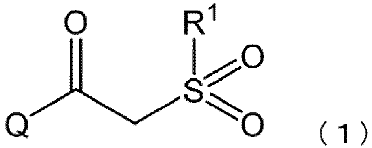
in formula (1), Q represents a substituted or unsubstituted 5- to 6-membered or 9- to 10-membered heteroaryl group, and R¹ represents a C1-6 alkyl group, 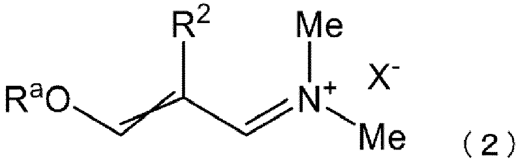
in formula (2), R² represents a hydrogen atom, a substituted or unsubstituted C1-6 alkyl group, a substituted or unsubstituted C3-6 cycloalkyl group, a substituted or unsubstituted phenyl group, or a substituted or unsubstituted 5- to 6-membered heteroaryl group, R^{a} represents a C1-6 alkyl group, and X⁻ represents a halide ion, 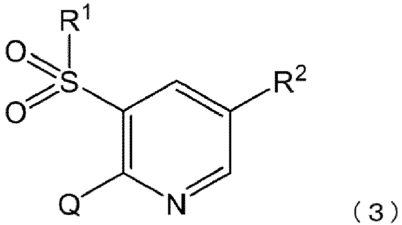
in formula (3), Q, R¹ and R² are the same as those in formula (1) and formula (2).

2. The method according to claim 1, wherein Q in formula (1) and formula (3) is a group represented by formula (4), 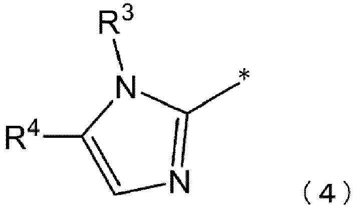 in formula (4), * represents a bonding site, R³ represents a C1-6 alkyl group, R⁴ represents a hydrogen atom, a substituted or unsubstituted C1-6 alkyl group, a substituted or unsubstituted C2-6 alkenyl group or a 1,3-dioxolan-2-yl group.

3. A compound represented by formula (1a), 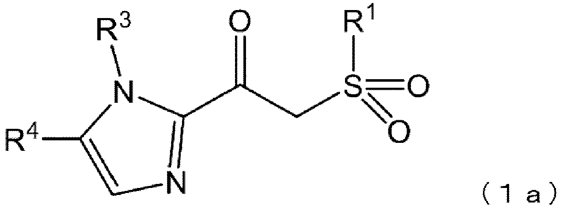 in formula (1a), R¹ represents a C1-6 alkyl group, R³ represents a C1-6 alkyl group, R⁴ represents a hydrogen atom, a substituted or unsubstituted C1-6 alkyl group, a substituted or unsubstituted C2-6 alkenyl group or a 1,3-dioxolan-2-yl group.
